# EUROPEAN PATENT APPLICATION

(11) **EP 4 628 201 A1**
(43) Date of publication of application: **08.10.2025**
(21) Application number: 23914920.6
(22) Date of filing: 27.10.2023
(51) Int. Cl.: B01F 33/30, B01F 25/00, B01L 3/00, B82Y 30/00, B82Y 40/00

(54) **SPIRAL-STRUCTURED CHIP FOR PREPARING LIPID NANOPARTICLES, AND METHOD FOR PREPARING LIPID NANOPARTICLES BY USING SAME**

(30) Priority: 04.01.2023 KR 20230001083; 26.10.2023 KR 20230145133
(71) Applicant: Inventage Lab Inc., Gyeonggi-do 13403 (KR)
(72) Inventor: LEE, Sang Hun, Seongnam-si, Gyeonggi-do 13494 (KR); SEO, Hee Won, Seongnam-si, Gyeonggi-do 13494 (KR); CHON, Chan Hee, Seongnam-si, Gyeonggi-do 13494 (KR); KIM, Dong Hoon, Seongnam-si, Gyeonggi-do 13494 (KR); KIM, Ju Hee, Seongnam-si, Gyeonggi-do 13494 (KR)
(74) Representative: BCKIP Part mbB
(86) International application number: PCT/KR2023/016813
(87) International publication number: WO 2024/147456

(57) **Abstract**

The present invention relates to a chip for producing lipid nanoparticles including a spiral structure and a method for producing lipid nanoparticles using the same. According to the present invention, it is possible to increase the efficiency of mixing between an aqueous-phase solution containing a nucleic acid and an oil-phase solution containing lipids, thereby forming uniform lipid nanoparticles by self-assembly at the interface between the aqueous-phase solution and the oil-phase solution. Moreover, the present invention relates to a production method capable of efficiently producing lipid nanoparticles having a uniform shape and diameter using the chip for producing lipid nanoparticles including a spiral structure.

## Description

### Technical Field

The present invention relates to a chip for producing lipid nanoparticles including a spiral structure and a method for producing lipid nanoparticles using the same.

### Background Art

Lipid nanoparticles (LNPs) are effective drug delivery systems for biologically active compounds such as therapeutic nucleic acids, proteins, and peptides, which are otherwise cell impermeable.

Commonly, vaccines are subdivided into "first generation", "second generation" and "third generation" vaccines. Genetic vaccines, that is, vaccines for genetic vaccination, are usually understood as "third generation" vaccines. Genetic vaccines are typically composed of genetically engineered nucleic acid molecules which allow expression of peptide or protein (antigen) fragments characteristic for a pathogen or a tumor antigen *in vivo.* Genetic vaccines are expressed upon administration to a patient after uptake by target cells. Expression of the administered nucleic acids results in production of the encoded proteins. In the case in which these proteins are recognized as foreign by the patient's immune system, an immune response is triggered.

DNA as well as RNA may be used as nucleic acid molecules for administration in the context of genetic vaccination. DNA is known to be relatively stable and easy to handle.

However, the use of DNA bears the risk of undesired insertion of the administered DNA fragments into the patient's genome potentially resulting in mutagenic events such as in loss of function of the impaired genes.

By using RNA instead of DNA for genetic vaccination, the risk of undesired genomic integration and generation of anti-DNA antibodies is minimized or avoided. However, RNA is a rather unstable molecular species which may readily be degraded by ubiquitous RNAses, and thus has problems of impermeability, fragility, and immunogenicity.

Although a lot of progress has been made over the past few years, lipid nanoparticle formulations have been used as an efficient method for mRNA vaccination capable of inducing adaptive immune responses.

The lipid nanoparticle formulations may improve *in vivo* nucleic acid delivery.

A drug delivery system using the lipid nanoparticles is a multi-component formulation including an ionizable lipid, a non-ionizable lipid, a neutral lipid, and a conjugated lipid. A cationic ionizable lipid binds to anionic nucleic acid, whereas other components support stable self-assembly of the lipid nanoparticles.

The lipid nanoparticles are produced in an optimal drug:lipid ratio, and protect nucleic acid from degradation and elimination in serum, are suitable for systemic or local delivery, and are capable of providing intracellular delivery of nucleic acid.

When the lipid nanoparticles are produced using a conventional production method, the size of the produced particles is not uniform, which causes a problem of reducing production efficiency, such as requiring a separate sorting process.

There is a need to develop a chip and a method for producing lipid nanoparticles, which may overcome this problem and may increase production efficiency by producing lipid nanoparticles with a uniform diameter.

### [Prior Art Documents]

### [Patent Document]

US 10,835,878

### DISCLOSURE

### Technical Problem

An object of the present invention is to provide a chip for producing lipid nanoparticles including a spiral structure and a method for producing lipid nanoparticles using the same.

Another object of the present invention is to provide a chip for producing lipid nanoparticles including a spiral structure, which may increase the efficiency of mixing between an aqueous-phase solution containing a nucleic acid and an oil-phase solution containing lipids, thereby forming uniform lipid nanoparticles by self-assembly at the interface between the aqueous-phase solution and the oil-phase solution.

Still another object of the present invention is to provide a production method capable of efficiently producing lipid nanoparticles having a uniform shape and diameter using the chip for producing lipid nanoparticles including a spiral structure.

### Technical Solution

In order to achieve the above object, the present invention relates to a chip for producing lipid nanoparticles, including: an oil-phase solution supply unit; an aqueous-phase solution supply unit; and a mixer unit including a spiral structure, wherein the mixer unit may be connected to the oil-phase solution supply unit and the aqueous-phase solution supply unit, and configured to mix an oil-phase solution supplied through the oil-phase solution supply unit with an aqueous-phase solution supplied through the aqueous-phase solution supply unit to form lipid nanoparticles.

In addition, the mixer unit may include ridges and valleys formed alternately along a spiral path on the circumferential surface thereof.

In addition, the cross-section of the mixer unit may have an outer diameter of 1 mm to 2 mm defined by the ridges and an inner diameter of 0.5 mm to 0.6 mm defined by the valleys.

In addition, the pitch of the spiral path on the circumferential surface of the mixer unit may be 1 mm to 3 mm.

In addition, the ridges and valleys of the mixer unit may have a depth of 0.1 mm to 0.5 mm.

In addition, the valleys formed along the spiral path on the circumferential surface of the mixer unit may have a width of 0.1 mm to 0.5 mm.

In addition, the mixer unit may include a mixing section for mixing the oil-phase solution and the aqueous-phase solution and a stabilization section for stabilizing the complete mixture of the oil-phase solution and the aqueous-phase solution, wherein the length ratio between the mixing section and the stabilization section may be 1: 1 to 1:5.

In addition, the mixer unit may be connected to a discharge unit, wherein the discharge unit may be connected to the other end of the mixer unit connected to the oil-phase solution supply unit and the aqueous-phase solution supply unit.

A method for producing lipid nanoparticles according to another embodiment of the present invention may include steps of: preparing an aqueous-phase solution containing a nucleic acid; preparing an oil-phase solution by dissolving lipids in an organic solvent; supplying the aqueous-phase solution and the oil-phase solution to an oil-phase solution supply unit and an aqueous-phase solution supply unit of a chip for producing lipid nanoparticles according to claim 1; and forming lipid nanoparticles by mixing the oil-phase solution and the aqueous-phase solution, supplied to the oil-phase solution supply unit and the aqueous-phase solution supply unit, in a mixer unit.

In addition, the oil-phase solution and the aqueous-phase solution may flow along a spiral path on the circumferential surface of the mixer unit and be mixed together to form a mixture solution, and the mixture solution may be stabilized while flowing along the spiral path.

In addition, the oil-phase solution and the aqueous-phase solution may form an interface therebetween along the spiral path on the circumferential surface of the mixer unit, and lipid nanoparticles may be formed by self-assembly between the lipids in the oil-phase solution and the nucleic acid in the aqueous-phase solution at the interface.

In addition, the oil-phase solution and the aqueous-phase solution may move through a mixing section in which they form the mixture solution while flowing through the mixer unit and a stabilization section in which the mixture solution is stabilized, and the length ratio between the mixing section and the stabilization section may be 1: 1 to 1:5.

In addition, the flow rate ratio between the oil-phase solution and the aqueous-phase solution, which are supplied to the oil-phase solution supply unit and the aqueous-phase solution supply unit, respectively, may be 1: 1 to 1:5.

In addition, the total flow rate of the oil-phase solution and the aqueous-phase solution may be 5 mL/min to 50 mL/min.

In addition, the method may further include, after the step of forming the lipid nanoparticles, a post-treatment step of diluting and filtering the lipid nanoparticles.

### Advantageous Effects

According to the present invention, it is possible to increase the efficiency of mixing between an aqueous-phase solution containing a nucleic acid and an oil-phase solution containing lipids, thereby forming uniform lipid nanoparticles by self-assembly at the interface between the aqueous-phase solution and the oil-phase solution.

In addition, it is possible to efficiently produce lipid nanoparticles having a uniform shape and diameter using the chip for producing lipid nanoparticles including a spiral structure.

### Brief Description of Drawings

FIG. 1 illustrates a conventional chip for producing lipid nanoparticles according to one embodiment of the present invention.
FIG. 2 illustrates a chip for producing lipid nanoparticles according to one embodiment of the present invention.
FIG. 3 illustrates the chip for producing lipid nanoparticles according to one embodiment of the present invention and an enlarged view of a portion where a mixer unit is coupled.
FIG. 4 illustrates the mixer unit of the chip for producing lipid nanoparticles according to one embodiment of the present invention.
FIG. 5 is a cross-sectional view of the mixer unit of the chip for producing lipid nanoparticles according to one embodiment of the present invention.
FIG. 6 shows a mixing section and a stabilization section in the mixer unit of the chip for producing lipid nanoparticles according to one embodiment of the present invention.
FIG. 7 shows the flows of an oil-phase solution and an aqueous-phase solution, which are injected into the mixer unit of the chip for producing lipid nanoparticles according to one embodiment of the present invention.
FIG. 8 shows the flows of an oil-phase solution and an aqueous-phase solution in the cross-section of the mixer unit of the chip for producing lipid nanoparticles according to one embodiment of the present invention.
FIG. 9 shows the results of an experiment on fluid flow in the mixer unit of the chip for producing lipid nanoparticles according to one embodiment of the present invention.
FIG. 10 shows the results of an experiment on fluid flow depending on changes in the total flow rate of an oil-phase solution and an aqueous-phase solution in the mixer unit of the chip for producing lipid nanoparticles according to one embodiment of the present invention.
FIG. 11 shows the results of an experiment on fluid flow in the mixer unit of the chip for producing lipid nanoparticles according to one embodiment of the present invention.
FIG. 12 shows the results of an experiment on fluid flow in the mixer unit of the chip for producing lipid nanoparticles according to one embodiment of the present invention.
FIG. 13 shows the results of an experiment on fluid flow in the mixer unit of the chip for producing lipid nanoparticles according to one embodiment of the present invention.
FIG. 14 shows the results of an experiment on fluid flow in the mixer unit of the chip for producing lipid nanoparticles according to one embodiment of the present invention.
FIG. 15 shows the results of an experiment on fluid flow in the mixer unit of the chip for producing lipid nanoparticles according to one embodiment of the present invention.

### Best Mode

The present invention relates to a chip for producing lipid nanoparticles, including: an oil-phase solution supply unit; an aqueous-phase solution supply unit; and a mixer unit including a spiral structure, wherein the mixer unit is connected to the oil-phase solution supply unit and the aqueous-phase solution supply unit, and is configured to mix an oil-phase solution supplied through the oil-phase solution supply unit with an aqueous-phase solution supplied through the aqueous-phase solution supply unit to form lipid nanoparticles.

### Mode for Invention

Hereinafter, embodiments of the present invention will be described in detail so that those skilled in the art may easily carry out the present invention. However, the present invention may be embodied in various different forms and is not limited to the embodiments described herein.

mRNA is an abbreviation for messenger ribonucleic acid. DNA with genetic information is transcribed into mRNA which is an intermediate that carries the genetic information from DNA to protein in the process of synthesizing protein using mRNA.

COVID-19 has focused attention on the development of mRNA vaccines. mRNA vaccines have several advantages over other types of vaccines. The biggest advantage of mRNA vaccines is that lipid nanoparticles (LNPs) containing mRNA correspond to platform technology, enabling rapid technology development against viruses with frequent mutations, such as COVID-19.

Specifically, mRNA may be produced by identifying a protective protein antigen and sequencing a gene for the antigen. When new mRNA is produced using such a method and the formulation design and production process of an existing mRNA vaccine is used, rapid mRNA vaccine production is possible. This means that, since mRNAs that encode different antigens are very similar chemically and physically, the formulation design and production process of a new mRNA vaccine may be performed in the same steps as the formulation design and production process of an existing mRNA vaccine.

Due to the negative charge of the phosphate group, mRNA is generally a polyanionic macromolecule in the pH range used for parenteral administration. Using the electrical properties of negatively charged mRNA as described above, lipid nanoparticles may be produced using a positively charged ionizable lipid (or cationic lipid). Specifically, an ionizable lipid is a positively charged lipid, and strongly binds to negatively charged mRNA through electrical attraction. In addition to the ionizable lipid, a non-ionizable lipid, a neutral lipid and a conjugated lipid are further included to form lipid nanoparticles.

US 9364435 B2 discloses a nucleic acid-lipid particle including (a) a nucleic acid, (b) a cationic lipid, (c) a non-cationic lipid, and (d) a conjugated lipid, wherein, based on the total lipid content in the particle, the cationic lipid is included in an amount of 50 mol% to 85 mol%, the non-cationic lipid is included in an amount of 13 mol% to 49.5 mol%, and the conjugated lipid is included in an amount of 0.5 mol% to 2 mol%.

In addition, EP 2279254 B1 discloses a nucleic acid-lipid particle including, based on the total lipid content in the particle, 50 mol% to 65 mol% of a cationic lipid, 49.5 mol% or less of a non-cationic lipid, 30 mol% to 40 mol% of cholesterol or a derivative thereof, and 0.5 mol% to 2 mol% of a conjugated lipid.

In order to produce lipid nanoparticles containing a nucleic acid as described above, an aqueous-phase solution containing a nucleic acid and an oil-phase solution containing lipids are used for the production, and a chip for producing lipid nanoparticles capable of mixing the aqueous-phase solution and the oil-phase solution is used.

The chip for producing the lipid nanoparticles is for mixing the aqueous-phase solution and the oil-phase solution by a microfluidic method, and lipid nanoparticles may be formed by self-assembly at the interface between the aqueous-phase solution and the oil-phase solution. Specifically, as described below, by mixing the flow of the aqueous-phase solution and the flow of the oil-phase solution, a mixture solution containing lipid nanoparticles may be produced by self-assembly of lipids and mRNA at the interface between the two fluids.

A conventional chip for producing the lipid nanoparticles may have a flow channel structure which has a two-dimensional curved channel and in which baffles are arranged alternately, as disclosed in WO 2018/190423.

The flow channel structure in which baffles are arranged alternately is characterized in that the dilution rate of the solution may be controlled by adjusting the width, length, and arrangement of the baffles, thereby controlling the diameter of the nanoparticles.

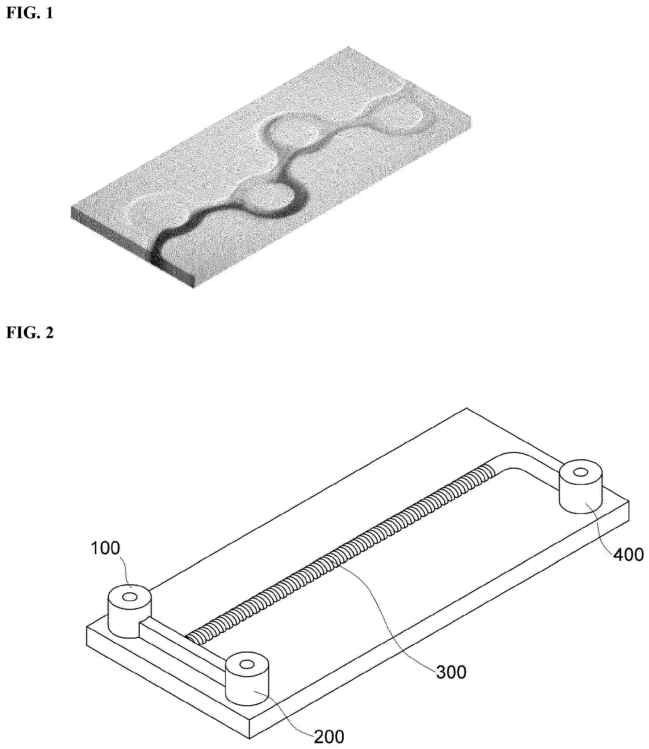

Alternatively, a method of using a fluidic mixer having bifurcated fluidic flow through toroidal mixing elements, as disclosed in KR 10-2361123 B1, has also been proposed. The fluidic mixer has a chip structure as illustrated in FIG. 1, and compared to the chip for producing lipid nanoparticles according to the present invention described later, it cannot achieve complete mixing of the oil-phase solution and the aqueous-phase solution, so shows a relatively low efficiency of production of lipid nanoparticles, shows a difference in the degree of uniformity of the particles.

That is, although various chips for producing lipid nanoparticles have been developed in the conventional art, conventional chips for producing lipid nanoparticles have a problem in that, even though the oil-phase solution and the aqueous-phase solution must be mixed together while forming an interface therebetween, mixing the oil-phase solution and the aqueous-phase solution is relatively difficult.

Accordingly, the present invention provides a chip for producing lipid nanoparticles including a novel spiral structure. When the chip for producing lipid nanoparticles including s spiral structure is used, it is possible to increase the efficiency of mixing between an aqueous-phase solution containing a nucleic acid and an oil-phase solution containing lipids, thereby forming uniform lipid nanoparticles by self-assembly at the interface between the aqueous-phase solution and the oil-phase solution.

Specifically, a chip for producing lipid nanoparticles according to one embodiment of the present invention includes: an oil-phase solution supply unit; an aqueous-phase solution supply unit; and a mixer unit including a spiral structure, wherein the mixer unit is may be connected to the oil-phase solution supply unit and the aqueous-phase solution supply unit, and configured to mix an oil-phase solution supplied through the oil-phase solution supply unit with an aqueous-phase solution supplied through the aqueous-solution supply unit to form lipid nanoparticles.

The mixer unit may be connected to a discharge unit, wherein the discharge unit may be connected to the other end of the mixer unit connected to the oil-phase solution supply unit and the aqueous-phase solution supply unit.

More specifically, the chip for producing lipid nanoparticles according to the present invention is as illustrated in FIG. 2. Referring to FIG. 2, the chip may include an oil-phase solution supply unit 100, an aqueous-phase solution supply unit 200, a mixer unit 300 including a spiral structure, and a discharge unit 400.

The oil-phase solution supply unit 100 and the aqueous-phase solution supply unit 200 may each be connected to one end of the mixer unit 300. The oil-phase solution and the aqueous-phase solution may be introduced into the mixer unit 300 through the oil-phase solution supply unit 100 and the aqueous-phase solution supply unit 200, and the oil-phase solution and the aqueous-phase solution may be mixed with each other within the mixer unit 300 to form lipid nanoparticles.

More specifically, in the chip for producing lipid nanoparticles according to the present invention illustrated in FIG. 2, the oil-phase solution supply unit 100 and the aqueous-phase solution supply unit 200 may be positioned with respect to the z-axis, extend vertically in the y-axis direction, and then be coupled to the mixer unit 300 extending in the x-axis direction.

The oil-phase solution supply unit 100 and the aqueous-phase solution supply unit 200 are equally positioned with respect to the z-axis so that the oil-phase solution and the aqueous-phase solution may be injected thereinto, respectively, and the oil-phase solution and the aqueous-phase solution may flow through the oil-phase solution supply unit 100 and the aqueous-phase solution supply unit 200. The oil-phase solution supply unit 100 and the aqueous-phase solution supply unit 200 are vertically connected to the mixer unit 300 at a portion where the flow of the oil-phase solution and the flow of the aqueous-phase solution meet each other, so that both the flow of the oil-phase solution and the flow of the aqueous-phase solution move into the interior of the mixer unit 300.

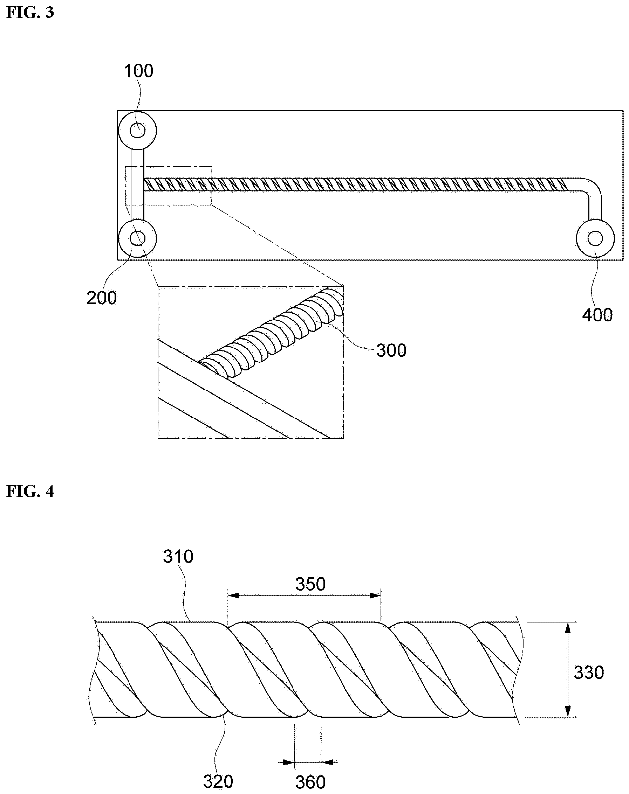

FIG. 3 shows the portion where the flow of the oil-phase solution and the flow of the aqueous-phase solution meet each and an enlarged view of the portion that is vertically connected to the mixer unit 300.

The flow of each of the oil-phase solution and the aqueous-phase solution and the flow of the mixture solution within the mixer unit 300 will be described later.

Referring to FIG. 3, the oil-phase solution supply unit 100 and the aqueous-phase solution supply unit 200 are in contact with each other in directions facing each other, and are vertically connected to the mixer unit 300 at a portion where the oil-phase solution supply unit 100 and the aqueous-phase solution supply unit 200 are in contact with each other.

The mixer unit 300 of the present invention is structurally characterized by including a spiral structure as described above, and specifically, the spiral structure is characterized by including ridges 310 and valleys 320 alternately formed along a spiral path on the circumferential surface thereof.

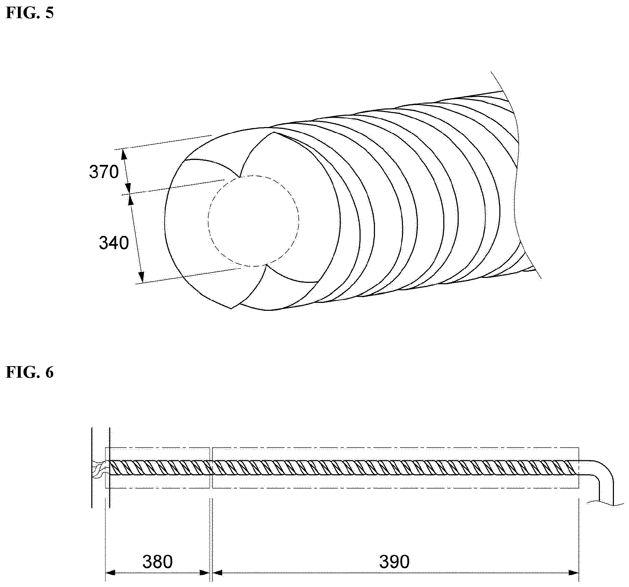

The spiral structure is characterized by having ridges 310 and valleys 320 repeatedly formed along a spiral path on the circumferential surface, and there is a difference between the outer diameter defined by the ridges 310 and the inner diameter defined by the valleys 320. That is, as shown in FIG. 5, a diameter 330 of the circle of the cross-section may be defined by the ridges 310, and the valleys 320 are concave portions along the spiral path. A diameter 340 of the circle of the cross-section may also be defined by the valleys 320.

The cross-section of the mixer unit 300 may have an outer diameter 330 of 1 mm to 2 mm defined by the ridges 310, and an inner diameter 340 of 0.5 mm to 0.6 mm defined by the valleys 320, or an outer diameter 330 of 1 mm to 1.8 mm defined by the ridges 310, and an inner diameter 340 of 0.51 mm to 0.59 mm defined by the valleys 320, or an outer diameter 330 of 1 mm to 1.5 mm defined by the ridges 310, and an inner diameter 340 of 0.52 mm to 0.58 mm defined by the valleys 320. As described below, when the flows of the oil-phase solution and the aqueous-phase solution occur within the mixer unit 300, the flow along the outer diameter 330 of the mixer unit 300 occurs, and the flow along the inner diameter 340 may also occur. That is, when the mixing of the oil-phase solution and the aqueous-phase solution within the mixer unit 300 occurs, the mixing may occur while the solutions flow along the outer diameter 330 of the mixer unit 300, and the flow along the inner diameter 340 may also occur, and as the flow along the outer diameter 330 and the flow along the inner diameter 340 are mixed with each other according to the movement of the fluids, a complete mixture of the solutions is formed. This means that the flows of the oil-phase solution and aqueous-phase solution injected into the mixer unit 300 of the present invention are not mixed with each other only by the flow caused by the shape of the spiral structure, but mixing occurs between the flow along the circumferential surface of the mixer unit 300 and the flow along a straight line in the center.

As shown in FIG. 4, the mixer unit 300 of the present invention may define a pitch 350 of the spiral path on the circumferential surface, the depth 370 of the ridges 310 and the valleys 320, and the width 360 of the valleys formed along the spiral path on the circumferential surface. Specifically, the pitch 350 of the spiral path on the circumferential surface may be 1 mm to 3 mm, the depth 370 of the ridges 310 and the valleys 320 may be 0.1 mm to 0.5 mm, and the width 360 of the valleys formed along the spiral path on the circumferential surface may be 0.1 mm to 0.5 mm. Alternatively, the pitch 350 of the spiral path on the circumferential surface may be 1.5 mm to 2.5 mm, the depth 370 of the ridges 310 and the valleys 320 may be 0.2 mm to 0.4 mm, and the width 360 of the valleys formed along the spiral path on the circumferential surface may be 0.2 mm to 0.4 mm.

The pitch 350 means the length from the starting point to the same point as the rotational movement along the circumferential surface from a specific point on the circumferential surface by the spiral structure. The depth 370 refers to the distance between the ridge 310 and the valley 320 in the cross-section of the mixer unit 300. The width 360 of the valleys refers to the repeating interval of the spiral structure, and the number of times the spiral structure is repeated within a specific section differs depending on the width 360 of the valleys.

Depending on the difference in the pitch 350, depth 370, and width 360 of the mixer unit 300 having the spiral structure as described above, the characteristics of the spiral structure change. When the characteristics of the spiral structure change, the flow of the fluid within the mixer unit 300 changes. Within the scope of the present invention with respect to the pitch 350, depth 370, and width 360 of the mixer unit 300 and the range of the length ratio between the mixing section and the stabilization section of the mixer unit 300 described below, uniform lipid nanoparticles may be formed by mixing the oil-phase solution and the aqueous-phase solution.

The mixer unit 300 includes a mixing section 380 for mixing the oil-phase solution and the aqueous-phase solution and a stabilization section 390 for stabilizing the complete mixture of the oil-phase solution and the aqueous-phase solution, and the length ratio between the mixing section 380 and the stabilization section 390 may be 1: 1 to 1:5.

Details regarding the flows of the fluids are shown in FIGS. 6 to 8.

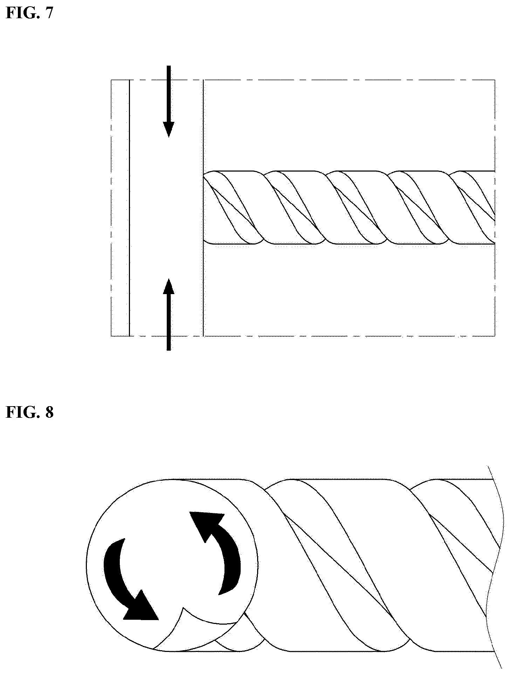

Specifically, referring to FIG. 7, when the oil-phase solution and the aqueous-phase solution are injected through the oil-phase solution supply unit 100 and the aqueous-phase solution supply unit 200 as described above, the oil-phase solution and the aqueous-phase solution flow in directions facing each other, and because the portion where the flow of the oil-phase solution and the flow of the aqueous-phase solution meet each other is connected to the mixer unit 300, the flow of the oil-phase solution and the flow of the aqueous-phase solution move to the mixer unit 300 while forming an interface therebetween.

The oil-phase solution and the aqueous-phase solution that have moved to the mixer unit 300 form an interface therebetween, and lipid nanoparticles are formed at the interface, and at the same time, the oil-phase solution and the aqueous-phase solution are mixed with each other to form a mixture solution. At this time, the efficiency of mixing between the oil-phase solution and the aqueous-phase solution may be further enhanced by the flows of fluids as shown in FIG. 8 within the mixer unit 300.

That is, the mixer unit 300 of the present invention is characterized by having the spiral structure as described above, and fluid flows occur along the periphery of the mixer unit 300 having the spiral structure. The fluid flows may increase the efficiency of mixing between the oil-phase solution and the aqueous-phase solution, and promote the formation of uniform lipid nanoparticles at the interface.

The oil-phase solution and the aqueous-phase solution mixed with each other while flowing along the mixer unit 300 pass through the mixing section 380 where the oil-phase solution and the aqueous-phase solution are mixed within the mixer unit 300 and a stabilization section 390 where the complete mixture of the oil-phase solution and the aqueous-phase solution is stabilized, thereby forming uniform lipid nanoparticles.

As described above, the oil-phase solution and the aqueous-phase solution are completely mixed with each other within the mixer unit 300 to form a mixture solution. When forming the mixture solution, the lipids in the oil-phase solution and the nucleic acid in the aqueous-phase solution form lipid nanoparticles by self-assembly, but in order to stabilize the lipid nanoparticles, a step of stabilizing the lipid nanoparticles in the flow of the complete mixture within the mixer unit 300 is performed.

The length ratio between the mixing section 380 and the stabilization section 390 may be 1:1 to 1:5, 1:1 to 1:4, or 1:2 to 1:4. Within the above length ratio range, the oil-phase solution and the aqueous-phase solution are completely mixed with each other, and then the complete mixture solution moves within the stabilization section. Within the above fluid flow, the formation of lipid nanoparticles and the stabilization of the formed lipid nanoparticles proceed simultaneously.

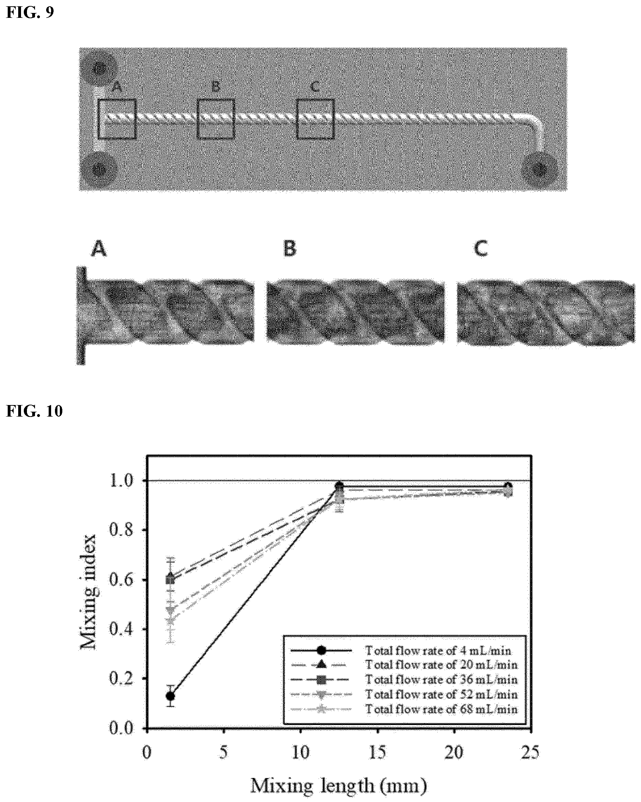

The mixing of the fluids will be described in more detail with reference to FIG. 9. FIG. 9 shows the results of confirming the mixing of the oil-phase solution and the aqueous-phase solution in each section of the mixer unit 300 in the chip for producing lipid nanoparticles according to the present invention by using dye solutions. A yellow solution was used instead of the oil-phase solution, and a blue solution was used instead of the aqueous-phase solution. When the solutions were completely mixed with each other, the mixing was confirmed through color change to a green solution.

Specifically, an experiment was conducted on flow at three locations shown in FIG. 9. As the working fluids used in the experiment, a solution prepared by diluting a yellow dye in deionized water at a concentration of 50 µM was used as the aqueous-phase solution, and a solution prepared by diluting a blue dye in ethanol at a concentration of 50 µM was used as the oil-phase solution. The contact surface between the two solutions within the mixer unit was photographed in a direction perpendicular to the top surface of the mixer unit. The intensity based on the pixels of the photographed image was analyzed through n-by-m pixels in each image by dividing the horizontal direction (x-axis) of the image by n and dividing the vertical direction (y-axis) by m. When the flow of fluid for each flow rate at the location to be measured was stabilized to a steady state in which it did not change over time, the photographs were taken. Additionally, for more accurate relative analysis, the photographs were taken when the oil-phase solution flowed and when only deionized water flowed. The case when only the oily solution flowed was considered as a value of 100% mixing, that is, a state in which complete mixing was achieved, and the case when only deionized water flowed was considered as a value of 0% mixing, a state in which mixing had not yet occurred.

Section A of FIG. 9 shows the mixing of the solutions at the beginning of the mixing section 300. Section A is the section where the yellow solution and the blue solution are mixed with each other. In section A, the yellow solution and the blue solution are found and a portion where the two solutions are mixed with each other to show a green color is also found.

Section B of FIG. 9 shows the degree of mixing of the two solutions that passed through section A. Compared to section A, a part showing a greener color is found, suggesting that a relatively complete mixture solution is further included.

Section C of FIG. 9 shows a stabilization section, and from the color, it can be seen that the mixture solution is in a completely mixed solution state.

On the other hand, FIG. 1 relates to the mixing of fluids in a chip for producing lipid nanoparticle disclosed in KR 10-2361123 B1. It can be confirmed that, after the oil-phase solution and the aqueous-phase solution are injected as disclosed in the present invention, complete mixing of the solutions within the entire section of the chip during the mixing process does not occur. Therefore, it can be confirmed that the degree of complete mixing of the oil-phase solution and the aqueous-phase solution differs from that in the chip for producing lipid nanoparticles according to the present invention.

When the chip for producing lipid nanoparticles shown in FIG. 1 is used, complete mixing of the oil-phase solution and the aqueous-phase solution is not achieved as described above, or toroidal mixing elements should be further included in the chip for complete mixing. That is, in the case where the oil-phase solution and the aqueous-phase solution are not completely mixed with each other, lipid nanoparticles are formed during mixing of the oil-phase solution and the aqueous-phase solution, but unlike the chip for producing lipid nanoparticles according to the present invention, the formed lipid nanoparticles do not pass through the stabilization section, and thus a problem may arise in that collisions may occur between the lipid nanoparticles or agglomeration may occur, making it difficult to produce uniform lipid nanoparticles. In addition, if toroidal mixing elements are further included for complete mixing, the chip size increases, which is not desirable in terms of cost.

A method for producing lipid nanoparticles according to another embodiment of the present invention may include steps of: (S100) preparing an aqueous-phase solution containing a nucleic acid; (S200) preparing an oil-phase solution by dissolving lipids in an organic solvent; (S300) supplying the aqueous-phase solution and the oil-phase solution to the oil-phase solution supply unit 100 and the aqueous-phase solution supply unit 200 of the above-described chip for producing lipid nanoparticles; and (S400) forming lipid nanoparticles by mixing the oil-phase solution and the aqueous-phase solution, supplied to the oil-phase solution supply unit 100 and the aqueous-phase solution supply unit 200, in the mixer unit 300.

Specifically, in step S100, an aqueous-phase solution is prepared by mixing a nucleic acid with a solvent. The solvent is a citric acid solution with a pH of 3.0, but is not limited to the above example, and any solvent capable of producing lipid nanoparticles by mixing the nucleic acid may be used without limitation.

The nucleic acid may be selected from the group consisting of RNA, DNA, short interfering RNA (siRNA), messenger RNA (mRNA), an aptamer, an antisense oligodeoxynucleotide (ODN), antisense RNA, ribozyme, DNAzyme, and mixtures thereof, and is preferably mRNA, but is not limited to the above examples.

The nucleic acid is used for preventing or treating a disease, and as an example, it is used to synthesize a spike protein to fight against the COVID-19 virus, such as a COVID-19 vaccine. Without being limited to the above example, any nucleic acid for preventing or treating a disease may be used.

In step S200, an oil-phase solution is prepared by dissolving an ionizable lipid, a non-ionizable lipid, a neutral lipid, and a conjugated lipid in an organic solvent.

The ionizable lipid may be ALC-0315 (Genevant), ALC-0159 (Genevant), DLinDAP, Dlin-MC3-DMA, or SM102 (Albutus). The ionizable lipid is not limited to the examples, and any ionizable lipid that may be used in the production of lipid nanoparticles may be used without limitation.

The non-ionizable lipid may be included together with the conjugated lipid to increase the stability of lipid nanoparticle. Lipid nanoparticles are intended to deliver nucleic acid to a target tissue or organ, but have the problem of being destroyed before reaching the target tissue or organ after injection into the body. To prevent this problem, the non-ionizable lipid and the conjugated lipid may be included. Specifically, the non-ionizable lipid is selected from the group consisting of distearoylphosphatidylcholine (DSPC), dioleolphosphatidyl ethanolamine (DOPE), bis(diphenylphosphino)ethane (DPPE), diacyl phosphatidylcholine, diacyl phosphatidylethanolamine, diacyl phosphatidylserine, and mixtures thereof, and is preferably DSPC, but is not limited to the above examples.

The conjugated lipid may be selected from the group consisting of cholesterol, tocopherol, and mixtures thereof, and is preferably cholesterol, but is not limited to the above examples.

The neutral lipid is included to control the size of the particles and act as a steric barrier to prevent agglomeration during storage. Specifically, it may be selected from the group consisting of polyethylene glycol 2000 distearoylphosphatidylethanolamine (PEG (2000) DSPE), DMG-PEG, PEG-DMPE, DPPE-PEG, DPG-PEG, PEG-DOPE, and mixtures thereof, and is preferably DMG-PEG, but is not limited to the above examples.

The organic solvent that is used to prepare the oil-phase solution is an alcohol. Specifically, it may be methanol, ethanol, isopropanol, n-propanol, or the like, and is preferably ethanol, but is not limited to the above examples, and any organic solvent capable of uniformly dissolving the ionizable lipid may be used without limitation.

In step S300, the aqueous-phase solution and the oil-phase phase solution prepared in steps S100 and S200 are injected into the oil-phase solution supply unit 100 and the aqueous-phase solution supply unit 200 and allowed to flow. As described above, the oil-phase solution and the aqueous-phase solution injected into the oil-phase solution supply unit 100 and the aqueous-phase solution supply unit 200, respectively, flow in directions facing each other, and because the point where the two flows meet each other is connected to the mixer unit 300, the two flows may form a laminar flow within the mixer unit 300 and be mixed with each other within the mixer unit 300.

In step S400, the aqueous-phase solution and the oil-phase solution injected into the mixer unit 300 may form a laminar flow at the point of intersection with the mixer unit and be mixed within the mixer unit 300, and the nucleic acid in the aqueous-phase solution and the ionizable lipid, the non-ionizable lipid, the neutral lipid, and the conjugated lipid in the oil-phase solution may be combined through self-assembly by electrostatic attraction to form lipid nanoparticles. That is, the lipid nanoparticles may be produced by mixing the aqueous-phase solution and the oil-phase solution, and the oil-phase solution may contain all of the four types of lipids.

The nucleic acid is specifically mRNA. As described above, mRNA is anionic, and the ionizable lipid is cationic, and a binding force may be generated by the electrostatic attraction between them, thereby forming lipid nanoparticles.

In step S400, the oil-phase solution and the aqueous-phase solution may be mixed with each other by flowing along a spiral path on the circumferential surface of the mixer unit 300, thereby forming a mixture solution. At this time, the oil-phase solution and the aqueous-phase solution pass through the mixing section 380 where they are mixed, and the stabilization section 390 where the mixture solution forming lipid nanoparticles that passed through the mixing section 380 is stabilized while flowing along the spiral path. That is, within the mixing section 380, the oil-phase solution and the aqueous-phase solution may form an interface therebetween along a spiral path on the circumferential surface of the mixer unit 300, and the lipids in the oil-phase solution and the nucleic acid in the aqueous-phase solution may form lipid nanoparticles by self-assembly at the interface. As described above, the interface formed between the oil-phase solution and the aqueous-phase solution during flowing along the spiral path on the circumferential surface of the mixer unit 300 may be formed by the flows shown in FIG. 8.

When the oil-phase solution and the aqueous-phase solution are supplied to the oil-phase solution supply unit and the aqueous-phase solution supply unit, respectively, the flow rate ratio therebetween may be 1:1 to 1:5, 1:1 to 1:4, or 1:2 to 1:4. When the solutions are mixed and used at a flow rate ratio within the above range, it is possible to produce lipid nanoparticles having a uniform particle size. More specifically, if the flow rate ratio is lower than the lower limit of the above range, there is a problem that it is difficult to maintain the shape of lipid nanoparticles due to the high content of ethanol during a subsequent process, and if the flow rate ratio is higher than the upper limit of the above range, there is a problem in that, since a large amount of the aqueous-phase solution is included, the movement of lipid particles in the aqueous-phase solution is restricted, so that particles with an excessively small diameter are formed.

The total flow rate of the oil-phase solution and the aqueous-phase solution may be 5 mL/min to 50 mL/min, 10 mL/min to 45 mL/min, or 15 mL/min to 40 mL/min. Within the above total flow rate range, the oil-phase solution and the aqueous-phase solution may be completely mixed with each other within a short time in the mixing section 380 of the mixer unit 300, so that uniform lipid nanoparticles may be produced.

FIG. 10 shows the results of conducting an experiment to determine the length of the mixing section 380 in the mixer unit 300 depending on the total flow rate of the oil-phase solution and the aqueous-phase solution of the present invention. It can be confirmed that, within the above range, a complete mixture solution is formed within a range where the length ratio between the mixing section 380 and the stabilization section 390 of the mixer unit 300 is 1:1 to 1:5.

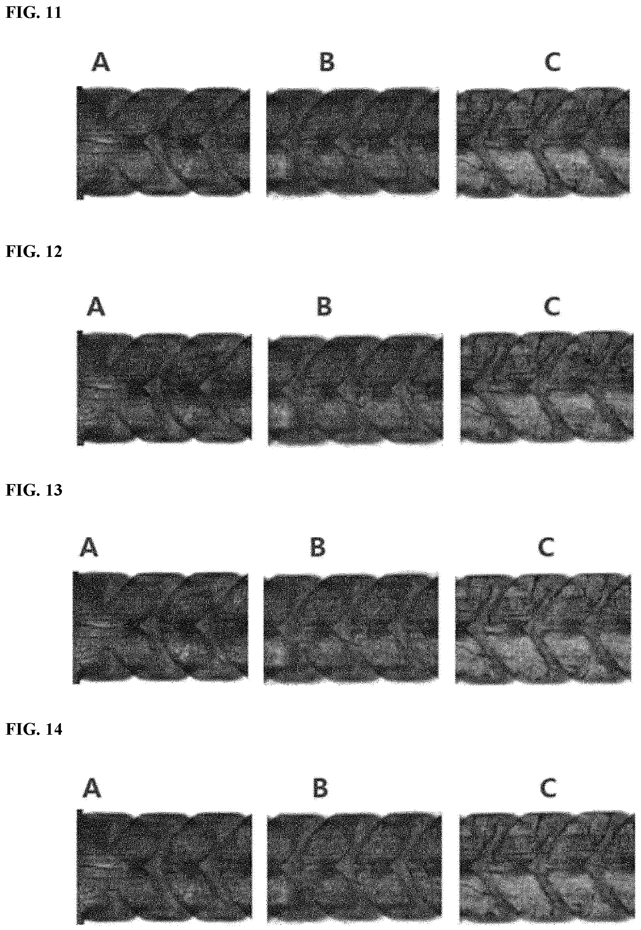

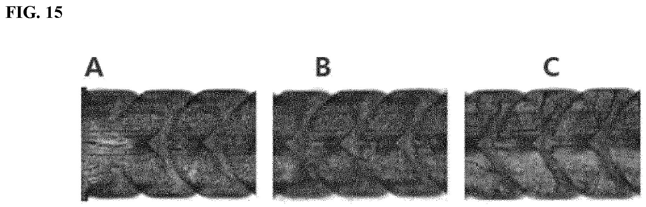

FIGS. 11 to 15 show the results of conducting an experiment on the flow in the same sections A to C as those in FIG. 9 while changing the total flow rate from 4 mL/min to 68 mL/min. As confirmed from the experimental results in FIG. 14, when the total flow rate of the oil-phase solution and the aqueous-phase solution is 4 mL/min, there is a problem that the formation of lipid nanoparticles in the mixture solution is not easy because the mixing index is significantly lowered within the section from 0 mm to 2 mm, which is the introduction section of the mixing section 300, until a complete mixture solution is formed.

In addition, even when the total flow rate of the oil-phase solution and the aqueous-phase solution is 52 mL/min or 68 mL/min, there is a problem in that the mixing speed of the oil-phase solution and the aqueous-phase solution in section A of the mixer unit 300 is relatively low, and thus the formation of lipid nanoparticles in the mixture solution is not easy.

On the other hand, it can be confirmed that, when the total flow rate of the oil-phase solution and the aqueous-phase solution is 20 mL/min or 36 mL/min, rapid mixing of the two solutions occurs in section A of the mixer unit 300, and mixing occurs quickly. In addition, because the section in which a complete mixture solution is formed is short, lipid nanoparticles having a uniform diameter may be formed within the mixing section.

The method may further include a post-treatment step of diluting and filtering the lipid nanoparticles formed in step S400.

The chip for producing lipid nanoparticles according to the present invention may be formed on a material selected from the group consisting of a glass substrate, a silicon wafer, or a polymer film; however, examples of the material are not limited to the above examples, and any material on which microchannels may be formed may be used.

The polymer film may be selected from the group consisting of polyimide, polyethylene, fluorinated ethylene propylene, polypropylene, polyethylene terephthalate, polyethylene naphthalate, polysulfone, and mixtures thereof, but is not limited to the above examples.

As an example, aluminum is deposited on a silicon wafer using an e-beam evaporator, and photoresist is patterned on the aluminum using a photolithography technique. Thereafter, the aluminum is etched using the photoresist as a mask, and the photoresist is removed. Then, the silicon is etched by DRIE (deep reactive ion etching) using the aluminum as a mask, and the aluminum is removed, and then glass is anodically bonded onto the wafer and hermetically sealed, thereby fabricating the chip.

Although the preferred embodiments of the present invention have been described in detail above, the scope of the present invention is not limited thereto, and various modifications and improvements made by those skilled in the art using the basic concept of the present invention defined in the following claims also fall within the scope of the present invention.

### Industrial Applicability

The present invention relates to a chip for producing lipid nanoparticles including a spiral structure and a method for producing lipid nanoparticles using the same.

## Claims

1. A chip for producing lipid nanoparticles, comprising:
an oil-phase solution supply unit;
an aqueous-phase solution supply unit; and
a mixer unit comprising a spiral structure,
wherein the mixer unit is connected to the oil-phase solution supply unit and the aqueous-phase solution supply unit, and is configured to mix an oil-phase solution supplied through the oil-phase solution supply unit with an aqueous-phase solution supplied through the aqueous-phase solution supply unit to form lipid nanoparticles.

2. The chip for producing lipid nanoparticles according to claim 1, wherein the mixer unit comprises ridges and valleys formed alternately along a spiral path on a circumferential surface thereof.

3. The chip for producing lipid nanoparticles according to claim 2, wherein a cross-section of the mixer unit has an outer diameter of 1 mm to 2 mm defined by the ridges and an inner diameter of 0.5 mm to 0.6 mm defined by the valleys.

4. The chip for producing lipid nanoparticles according to claim 1, wherein a pitch of a spiral path on a circumferential surface of the mixer unit is 1 mm to 3 mm.

5. The chip for producing lipid nanoparticles according to claim 2, wherein the ridges and valleys of the mixer unit have a depth of 0.1 mm to 0.5 mm.

6. The chip for producing lipid nanoparticles according to claim 2, wherein the valleys formed along the spiral path on the circumferential surface of the mixer unit have a width of 0.1 mm to 0.5 mm.

7. The chip for producing lipid nanoparticles according to claim 1, wherein the mixer unit comprises a mixing section for mixing the oil-phase solution and the aqueous-phase solution and a stabilization section for stabilizing a complete mixture of the oil-phase solution and the aqueous-phase solution,
wherein a length ratio between the mixing section and the stabilization section is 1:1 to 1:5.

8. The chip for producing lipid nanoparticles according to claim 1, wherein the mixer unit is connected to a discharge unit,
wherein the discharge unit is connected to the other end of the mixer unit connected to the oil-phase solution supply unit and the aqueous-phase solution supply unit.

9. A method for producing lipid nanoparticles, comprising steps of:
preparing an aqueous-phase solution containing a nucleic acid;
preparing an oil-phase solution by dissolving lipids in an organic solvent;
supplying the aqueous-phase solution and the oil-phase solution to the oil-phase solution supply unit and the aqueous-phase solution supply unit of the chip for producing lipid nanoparticles according to claim 1; and
forming lipid nanoparticles by mixing the oil-phase solution and the aqueous-phase solution, supplied to the oil-phase solution supply unit and the aqueous-phase solution supply unit, in the mixer unit.

10. The method for producing lipid nanoparticles according to claim 9, wherein the oil-phase solution and the aqueous-phase solution flow along a spiral path on a circumferential surface of the mixer unit and are mixed with each other to form a mixture solution, and the mixture solution is stabilized while flowing along the spiral path.

11. The method for producing lipid nanoparticles according to claim 10, wherein the oil-phase solution and the aqueous-phase solution form an interface therebetween along the spiral path on the circumferential surface of the mixer unit, and lipid nanoparticles are formed by self-assembly between the lipids in the oil-phase solution and the nucleic acid in the aqueous-phase solution at the interface.

12. The method for producing lipid nanoparticles according to claim 10, wherein the oil-phase solution and the aqueous-phase solution move through a mixing section in which they form the mixture solution while flowing through the mixer unit and a stabilization section in which the mixture solution is stabilized, wherein a length ratio between the mixing section and the stabilization section is 1: 1 to 1:5.

13. The method for producing lipid nanoparticles according to claim 9, wherein a flow rate ratio between the oil-phase solution and the aqueous-phase solution, which are supplied to the oil-phase solution supply unit and the aqueous-phase solution supply unit, respectively, is 1: 1 to 1:5.

14. The method for producing lipid nanoparticles according to claim 9, wherein a total flow rate of the oil-phase solution and the aqueous-phase solution is 5 mL/min to 50 mL/min.

15. The method for producing lipid nanoparticles according to claim 9, further comprising, after the step of forming the lipid nanoparticles, a post-treatment step of diluting and filtering the lipid nanoparticles.
